# EUROPEAN PATENT APPLICATION

(11) **EP 2 051 067 A1**
(43) Date of publication of application: **22.04.2009**
(21) Application number: 07767353.1
(22) Date of filing: 21.06.2007
(51) Int. Cl.: G01N 27/12

(54) **HYDROGEN GAS CONCENTRATION SENSOR AND APPARATUS FOR DETERMINING HYDROGEN GAS CONCENTRATION**

(30) Priority: 11.08.2006 JP 2006219832
(71) Applicant: KABUSHIKI KAISHA ATSUMITEC, Hamamatsu-shi Shizuoka 431-0192 (JP)
(72) Inventor: UCHIYAMA, Naoki, Hamamatsu-shi, Shizuoka 431-0192 (JP); KANAI, Tomomi, Hamamatsu-shi, Shizuoka 431-0192 (JP)
(74) Representative: Burt, Matthew Thomas
(86) International application number: PCT/JP2007/062527
(87) International publication number: WO 2008/018243

(57) **Abstract**

A hydrogen-gas concentration sensor comprises a substrate, and a plurality of hydrogen detecting films formed on the substrate, adjacent to one another. The hydrogen detecting films have a thin film layer, and a catalyst layer formed on the thin film layer. Each catalyst layer, when in contact with a hydrogen gas, exerts photocatalysis to hydrogenate each thin film layer reversibly and causes the electric resistance value thereof to change reversibly. The individual thin film layers have different sensitivities of a change in the hydrogen gas concentration vs. a change in the resistance value and different hydrogen gas concentration measurement ranges. The hydrogen-gas concentration sensor measures the hydrogen gas concentration with a thin film layer having a high sensitivity when the hydrogen gas concentration is low, and measures the hydrogen gas concentration with a thin film layer having a wide measurement range when the hydrogen gas concentration is high.

## Description

### Technical Field

The present invention relates to a hydrogen-gas concentration sensor and hydrogen-gas concentration measuring device for measuring a hydrogen gas concentration.

### Background Art

Measurement of a hydrogen gas concentration is essential in a manufacturing process of hydrogen gas, monitoring of the operational state of a fuel cell system, or the like. It is also essential in a hydrogen gas manufacturing plant, a hydrogen gas storage facility and so forth from the viewpoint of safety control. In this respect, a technique relating to a hydrogen absorption alloy or the like which selectively absorbs hydrogen gas and whose electric resistance value (hereinafter "resistance value") changes reversibly is developed, and such a technique is disclosed in, for example, Japanese Unexamined Patent Publication No. 2005-256028. A hydrogen-gas concentration measuring technique using photocatalysis, i.e., a technique relating to a thin film layer or the like whose resistance value changes reversibly when in contact with a sample gas that is oxidized and decomposed by a photocatalyst layer. Such a technique is disclosed in, for example, Japanese Unexamined Patent Publication No. 2005-214933. Those techniques need not use an electrolyte under normal temperature. Those techniques can realize a hydrogen-gas concentration sensor and a hydrogen-gas concentration measuring device which can be downsized and made lighter.

However, measurement of the hydrogen gas concentration based on a change in the resistance value of a hydrogen absorption alloy depends on how much hydrogen the hydrogen absorption alloy can absorb and how much the resistance value changes (i.e., the variation range of the resistance value, which is the difference between the resistance value when hydrogen is not absorbed at all and the resistance value when the resistance value has changed to its limit with hydrogen absorbed). Therefore, the measurement range of the hydrogen gas concentration (hereinafter "measurement range") is limited. There also is a similar limit in the technique of changing the resistance value of a thin film layer reversibly by oxidizing and decomposing a sample gas with a photocatalyst layer.

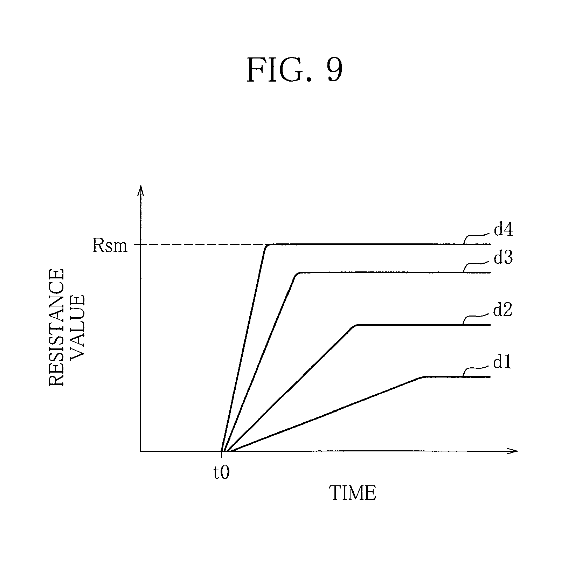

Fig. 9 is a graph showing the change-in-resistance-value characteristic of a hydrogen-gas concentration sensor having a photocatalyst layer and a thin film layer. Fig. 9 shows how the resistance value of the hydrogen-gas concentration sensor which has been kept in contact with a hydrogen gas since time t0 changes with the elapse of time, with the hydrogen gas concentration used as a parameter. Here, d1 to d4 indicate hydrogen gas concentrations, and the hydrogen gas concentration d1 is the lowest while the hydrogen gas concentration becomes higher in the order of d2, d3, and d4.

When the hydrogen gas concentration is low, the resistance value of a thin film layer increases comparatively slowly, and reaches to a steady state of a low resistance value. As the hydrogen gas concentration becomes higher, the resistance value of a thin film layer increases faster, and reaches the steady state of a higher resistance value. If the hydrogen gas concentration exceeds a certain limit, however, the resistance value of the thin film in the steady state reaches a ceiling resistance value Rsm (Fig. 9 shows that the resistance value of a hydrogen-gas concentration sensor reaches the ceiling resistance value Rsm at the concentration d4), and does not rise further. It is not therefore possible to measure the hydrogen gas concentration equal to or higher than d4 (because the hydrogen gas concentration exceeds the upper limit of the measurement range). Therefore, the use of a hydrogen-gas concentration sensor with a wide variable range of the resistance value makes the measurement range wider to ensure measurement of higher concentrations, but reduces the measuring accuracy in a low concentration area. On the other hand, the use of a hydrogen-gas concentration sensor with a narrow variable range of the resistance value can ensure highly accurate measurement in a low concentration area, but cannot ensure measurement of high concentrations because of the narrow measurement range.

The conventional hydrogen-gas concentration measuring techniques apparently have a problem that the high accuracy of measurement cannot be maintained over a wide measurement range.

### Disclosure of the Invention

It is an object of the present invention to provide a hydrogen-gas concentration sensor and hydrogen-gas concentration measuring device which can keep high measuring accuracy over a wide measurement range. Preferably, an additional object of the present invention is to provide a hydrogen-gas concentration measuring device which can find an abnormality in a hydrogen-gas concentration sensor or the hydrogen-gas concentration measuring device.

To achieve the object or the objects, a hydrogen-gas concentration sensor according to the present invention comprises a substrate, and a plurality of hydrogen detecting films formed on the substrate, adjacent to one another. Further, each of the plurality of hydrogen detecting films has a thin film layer formed on the substrate, and a catalyst layer formed on a surface of the thin film layer. When each of the hydrogen detecting films contacts a hydrogen gas contained in an atmosphere (i.e., air to be subjected to hydrogen-gas concentration measurement), the catalyst layer of each of the hydrogen detecting films exerts photocatalysis to hydrogenate the thin film layer reversibly. When the respective thin film layers are hydrogenated, electric resistance values of the respective thin film layers change reversibly according to the hydrogen gas concentration in the atmosphere. The change characteristics of the resistance values of the thin film layers (which are sensitivities to detect a change in hydrogen gas concentration as a change in resistance value or hydrogen-gas concentration measuring sensitivities) differ from one another.

The hydrogen gas concentrations in the atmospheres which are in contact with the individual hydrogen detecting films formed adjacent to one another can be regarded as substantially the same concentration. When the hydrogen gas concentration is low (i.e., when the resistance value has not reached the ceiling resistance value in any of the hydrogen detecting films), therefore, the hydrogen-gas concentration sensor can measure the hydrogen gas concentration with high accuracy by measuring the resistance value of a thin film layer which has a large change in resistance value (i.e., a high sensitivity) with respect to the hydrogen gas concentration. When the hydrogen gas concentration is high, the hydrogen-gas concentration sensor can measure the hydrogen gas concentration over a wide measurement range by measuring the resistance values of other thin film layers than the thin film layer whose resistance value has changed to the ceiling resistance value. In this manner, the hydrogen-gas concentration sensor according to the invention can measure the hydrogen gas concentration with high accuracy over a wide range.

Specifically, in the foregoing hydrogen-gas concentration sensor, the thin film layer in each hydrogen detecting film layer, for example, may be formed by a magnesium nickel alloy thin film layer or a magnesium thin film layer, and the catalyst layer may be formed of palladium or platinum.

A hydrogen-gas concentration measuring device according to the invention comprising a hydrogen-gas concentration sensor for measuring a hydrogen gas concentration making use of photocatalysis, a light source for irradiating the hydrogen-gas concentration sensor with light, and a data processing unit for measuring a hydrogen gas concentration using the hydrogen-gas concentration sensor. The hydrogen-gas concentration sensor is configured as described above. The data processing unit comprises a resistance measuring section for measuring the resistance value of each of the thin film layers of the plurality of hydrogen detecting films of the hydrogen-gas concentration sensor, and a measurement controlling section for measuring a hydrogen gas concentration on the basis of the resistance values of the thin film layers which are measured by the resistance measuring section. When none of the resistance values of the hydrogenated thin film layers have reached a predetermined limit resistance value (i.e., when the hydrogen gas concentration is low), the measurement controlling section measures a hydrogen gas concentration on the basis of the electric resistance value of the thin film layer which has a largest change in resistance value with respect to the hydrogen gas concentration. The limit resistance value will be described in detail in the description of an embodiment. On the other hand, when the thin film layers include a thin film layer whose electric resistance value has reached the predetermined limit resistance value, the measurement controlling section measures the hydrogen gas concentration on the basis of the electric resistance value of a thin film layer whose electric resistance value has not reached the limit resistance.

When the hydrogen gas concentration is low, therefore, the hydrogen-gas concentration measuring device can measure the hydrogen gas concentration on the basis of the resistance value of a thin film layer which has the highest sensitivity with high accuracy. When the thin film layers include a thin film layer whose resistance value has reached the limit resistance value, the hydrogen-gas concentration measuring device can measure the hydrogen gas concentration on the basis of the resistance value of a thin film layer whose electric resistance value has not reached the limit resistance value. Accordingly, the hydrogen-gas concentration measuring device according to the present invention can enlarge the measurement range for the hydrogen gas concentration, and can measure the hydrogen gas concentration with high accuracy over a wide range.

Preferably, when the hydrogenated thin film layers include a thin film layer whose electric resistance value has reached the predetermined limit resistance value, the hydrogen-gas concentration measuring device may measure a hydrogen gas concentration on the basis of the electric resistance value of a thin film layer that has a highest sensitivity among those thin film layers whose resistance values have not reached the limit resistance value. This can keep the highest measuring accuracy for the hydrogen gas concentration.

In the hydrogen-gas concentration sensor according to the invention, a plurality of hydrogen detecting films contact hydrogen gases with substantially the same concentration. Therefore, preferably, when the hydrogen-gas concentration measuring device detects a hydrogen gas, the resistance measuring section may measure a variation per unit time in the electric resistance value of each of the thin film layers, and the measurement controlling section may compare the variations per unit time in the resistance values of at least two thin film layers with each other to acquire a value corresponding to a comparison result. In this case, it is possible to determine that the measurement result of the hydrogen-gas concentration sensor and/or the hydrogen-gas concentration measuring device has an abnormality when the value corresponding to the comparison result exceeds a predetermined range. It is therefore possible to promptly detect a failure of a hydrogen-gas concentration sensor or a hydrogen-gas concentration measuring device.

As described above, the present invention can provide a hydrogen-gas concentration sensor and hydrogen-gas concentration measuring device which can maintain a high measuring accuracy over a wide measurement range making use of photocatalysis.

### Brief Description of the Drawings

Fig. 1 is a plan view showing the schematic configuration of a hydrogen-gas concentration sensor according to one embodiment of the present invention;
Fig. 2 is a cross-sectional view showing the schematic configuration of the hydrogen-gas concentration sensor shown in Fig. 1;
Fig. 3 is a graph showing a change in unit time (dt) in the resistance value of each thin film layer when a hydrogen gas contacts each hydrogen detecting film of the hydrogen-gas concentration sensor shown in Fig. 1;
Fig. 4 is a schematic configuration diagram of a hydrogen-gas concentration measuring device according to one embodiment of the present invention;
Fig. 5 is a flowchart of a hydrogen-gas concentration measurement performed by the hydrogen-gas concentration measuring device shown in Fig. 4;
Fig. 6A is a graph showing the relationship between the resistance value of each thin film layer and limit resistance value in the hydrogen-gas concentration measuring device shown in Fig. 4 when the hydrogen gas concentration is low;
Fig. 6B is a graph showing the relationship between the resistance value of each thin film layer and limit resistance value in the hydrogen-gas concentration measuring device shown in Fig. 4 when the hydrogen gas concentration is an intermediate concentration;
Fig. 6C is a graph showing the relationship between the resistance value of each thin film layer and limit resistance value in the hydrogen-gas concentration measuring device shown in Fig. 4 when the hydrogen gas concentration is high;
Fig. 7 is a graph for explaining the measurement range of the hydrogen-gas concentration measuring device shown in Fig. 4;
Fig. 8 is a flowchart of detection of an abnormality in the hydrogen gas concentration sensor or hydrogen-gas concentration measuring device performed by the hydrogen-gas concentration measuring device shown in Fig. 4; and
Fig. 9 is a graph showing the change-in-resistance-value characteristic of the conventional hydrogen-gas concentration sensor along with the relationship between the hydrogen gas concentration and ceiling resistance value.

### Best Mode of Carrying Out the Invention

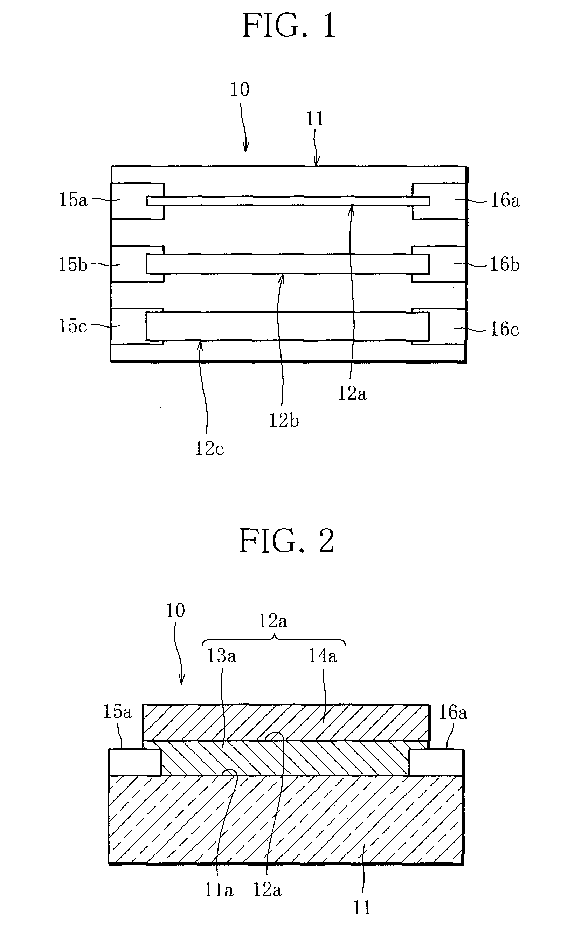

With reference to Figs. 1 to 8, a hydrogen-gas concentration sensor and hydrogen-gas concentration measuring device according to one embodiment of the present invention will be described below.

First, configuration of a hydrogen-gas concentration sensor 10 according to the embodiment will be described referring to Figs. 1 to 3. Fig. 1 is a plan view showing the schematic configuration of the hydrogen-gas concentration sensor according to one embodiment of the invention, and Fig. 2 is a cross-sectional view showing the schematic configuration of the hydrogen-gas concentration sensor.

As shown in Fig. 1, the hydrogen-gas concentration sensor 10 has a substrate 11 formed of a metal, glass, acrylic resin or the like, and a first hydrogen detecting film 12a, a second hydrogen detecting film 12b and a third hydrogen detecting film 12c which are formed on the substrates 11.

As shown in Fig. 2, the first hydrogen detecting film 12a has a thin film layer 13a formed on the surface of the substrate 11, and a catalyst layer 14a formed on the surface of the thin film layer 13a. A first electrode 15a is connected to one end of the thin film layer 13a, and a second electrode 16a is connected to the other end of the thin film layer 13a.

The second hydrogen detecting film 12b, like the first hydrogen detecting film 12a, has a thin film layer 13b and a catalyst layer 14b. A first electrode 15b is connected to one end of the thin film layer 13b, and a second electrode 16b is connected to the other end of the thin film layer 13b (Fig. 1).

The third hydrogen detecting film 12c, like the first hydrogen detecting film 12a, has a thin film layer 13c and a catalyst layer 14c. A first electrode 15c is connected to one end of the thin film layer 13c, and a second electrode 16c is connected to the other end of the thin film layer 13c (Fig. 1).

Although the thin film layers 13a to 13c is made of the same component and has the same length, the width of the thin film layer 13a is narrower than the width of the thin film layer 13b whose width is narrower than the width of the thin film layer 13c. The catalyst layer 14a, the catalyst layer 14b, and the catalyst layer 14c are formed in correspondence to the shapes of the thin film layer 13a, the thin film layer 13b, and the thin film layer 13c, respectively. The thin film layers 13a to 13c can be formed by sputtering, vacuum deposition, electron beam deposition, plating, etc., and their compositions are MgNix (0≤x<0.6), for example. The catalyst layers 14a to 14c can be formed on the surfaces of the respective thin film layers by coating or the like, with a thickness of 1 nm to 100 nm, for example.

With these thin film layers 13a to 13c and the catalyst layers 14a to 14c being formed, when the hydrogen-gas concentration sensor 10 contacts the atmosphere whose hydrogen concentration is about 100 ppm or higher, the resistance values of the thin film layers 13a to 13c change promptly within a time of 10 or more milliseconds, for example (resistance value becomes high).

Next, the operation of the thus configured hydrogen-gas concentration sensor 10 will be described below.

With being illuminated by light from a light source, when a hydrogen gas contacts the first hydrogen detecting film 12a, the second hydrogen detecting film 12b, and the third hydrogen detecting film 12c that the hydrogen-gas concentration sensor 10 has, the catalyst layers 14a to 14c exert the photocatalysis to hydrogenate the thin film layers 13a to 13c. Accordingly, the resistance values of the thin film layers 13a to 13c increase with time, and reach a steady state.

It is assumed that with the atmosphere (air) whose hydrogen gas concentration is d (ppm) being in contact with the hydrogen-gas concentration sensor 10, the resistance value in the steady state of the thin film layer 13a is Rad, the resistance value in the steady state of the thin film layer 13b is Rbd, and the resistance value in the steady state of the thin film layer 13c is Rcd. In the hydrogen-gas concentration sensor 10 of the present embodiment, the thin film layers 13a to 13c are formed so as to meet an equation Rad=2·Rbd=4·Rcd. That is, the first hydrogen detecting film 12a has a measuring sensitivity for hydrogen gas concentration twice as high as that of the second hydrogen detecting film 12b whose measuring sensitivity for hydrogen gas concentration is twice as high as that of the third hydrogen detecting film 12c. It is noted that the relationship among the resistance values Rad, Rbd and Rcd in the hydrogen-gas concentration sensor 10 is not limited to the aforementioned proportionality, as long as the relationship Rad>Rbd>Rcd is satisfied.

If the ceiling values of the resistance values of the hydrogenated thin film layers 13a to 13c are set to the resistance values Ram, Rbm and Rcm, respectively, the hydrogen detecting films 12a to 12c are formed so that the resistance value Ram is slightly higher than the resistance value Rbm, and the resistance value Rbm is slightly higher than the resistance value Rcm. Provided that with the concentration of the hydrogen gas being 0 (ppm), the resistance value of the thin film layer 13a is Ra0, the resistance value of the thin film layer 13b is Rb0 and the resistance value of the thin film layer 13c is Rc0, the resistance values Ra0, Rb0, and Rc0 are significantly smaller than the resistance values Ram, Rbm, and Rcm, respectively. Therefore, the variation ranges of the resistance values of the thin film layers 13a to 13c are approximately identical.

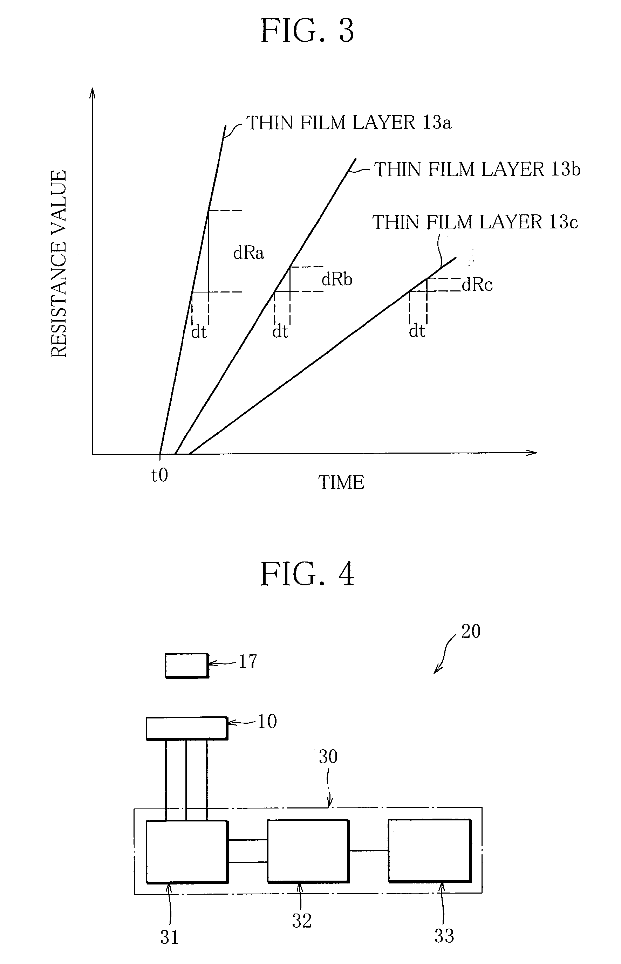

Fig. 3 is a graph showing changes in the resistance values of the thin film layers 13a to 13c when a hydrogen gas contacts the hydrogen-gas concentration sensor 10. As shown in Fig. 3, when the hydrogen gas of the concentration d (ppm) keeps contacting the hydrogen-gas concentration sensor 10 from the time t0, the resistance values of the thin film layers 13a to 13c become higher with the elapse of time. Provided that changes in the resistance value of the thin film layers 13a to 13c per unit time (dt) before the resistance values of the thin film layers 13a to 13c reach the steady values (the resistance values Rad, Rbd and Rcd) are dRa, dRb and dRc respectively, the relation dRa=2·dRb=4·dRc is satisfied in the hydrogen-gas concentration sensor 10 of the present embodiment. It is noted that the relationship among dRa, dRb and dRc is not limited to the aforementioned proportionality, as long as the relationship dRa>dRb>dRc is satisfied.

Because of differences in the reaction times of the thin film layers 13a to 13c with respect to the photocatalysis, the resistance value of the thin film layer 13b starts increasing with a slight delay from that of the thin film layer 13a, and the resistance value of the thin film layer 13c starts increasing with a slight delay from that of the thin film layer 13b.

Next, a hydrogen-gas concentration measuring device according to one embodiment of the present invention will be described referring to Fig. 4. As shown in Fig. 4, a hydrogen-gas concentration measuring device 20 has the aforementioned hydrogen-gas concentration sensor 10, a light source 17 which irradiates the hydrogen-gas concentration sensor 10 with light, and a data processing unit 30. With the hydrogen-gas concentration sensor 10 and the light source 17 being covered with a box or the like, as needed, to eliminate the influence of the outdoor light, the hydrogen-gas concentration measuring device may measure the hydrogen gas concentration in atmosphere that is circulated into this box.

The data processing unit 30 has a resistance measuring section 31 which measures the resistance values of the thin film layers 13a to 13c of the hydrogen detecting films 12a to 12c which the hydrogen-gas concentration sensor 10 has, a measurement controlling section 32 which controls the operation of the resistance measuring section 31 and processes measured data from the resistance measuring section 31, and a display section 33 which displays data or the like of the hydrogen gas concentration processed by the measurement controlling section 32.

The resistance value measuring section 31 supplies a predetermined current to the thin film layer 13a to measure a voltage drop between the first electrode 15a and the second electrode 16a. On the basis of the voltage drop and the value of the current, the resistance value measuring section 31 calculates the resistance value of the thin film layer 13a. The calculation of the resistance value is performed on the basis of the voltage drop and current value subjected to analog-to-digital conversion. The calculated resistance value is sent to the measurement controlling section 32 as digital data. The resistance values of the thin film layers 13b and 13c are likewise calculated and are sent to the measurement controlling section 32 by the resistance value measuring section 31.

The measurement controlling section 32 has, for example, a microprocessor and a memory device storing a program for the microprocessor. The measurement controlling section 32 controls the resistance value measuring section 31 such that the resistance value measuring section 31 measures the resistance values of the thin film layers 13a to 13c every unit time (e.g., dt (seconds)). The measurement controlling section 32 can record the measured data or the like obtained from the resistance value measuring section 31, and display the hydrogen gas concentration or the like on the display section 33 in a predetermined form.

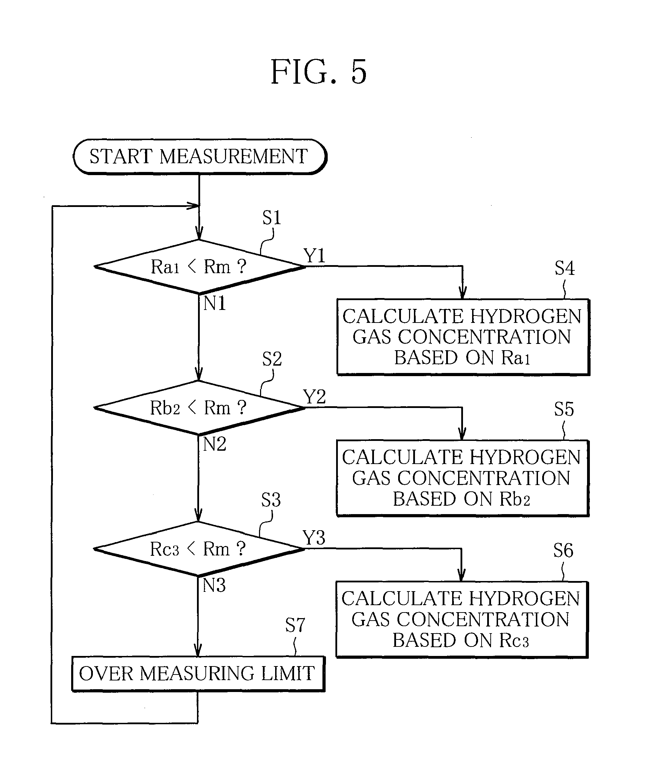

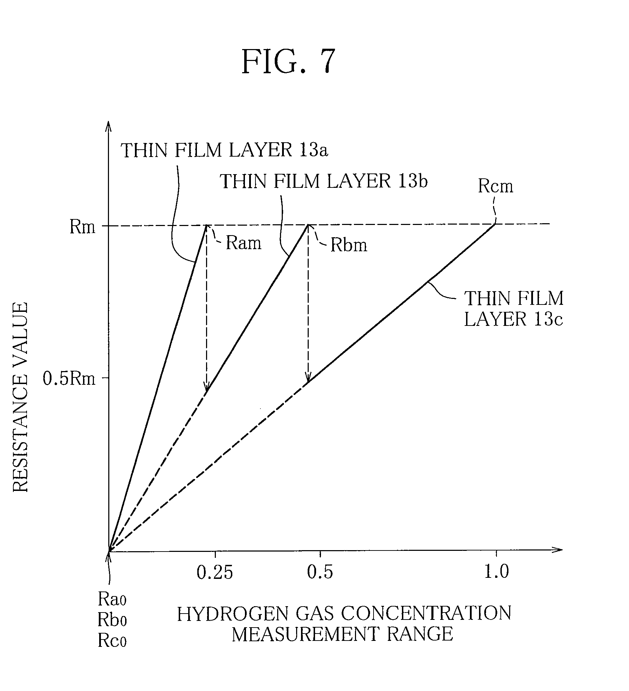

Next, the hydrogen-gas concentration measurement in the hydrogen-gas concentration measuring device 20 will be explained referring to Figs. 5 and 7. The hydrogen-gas concentration measuring device 20 has the upper limits of the hydrogen-gas concentration measurement ranges of the thin film layers 13a to 13c (upper limit values of resistance values) specified for the respective thin film layers 13a to 13c in consideration of variations in the resistance values Ram, Rbm and Rcm which are ceiling values of the resistance values of the thin film layers 13a to 13c.

Specifically, the lowest resistance value among the resistance values Ram, Rbm and Rcm, or a resistance value slightly lower than the lowest resistance value is set as a limit resistance value Rm. According to the embodiment, the thin film layer 13a is used in the variation range of the resistance values Ra0 through Rm, the thin film layer 13b is used in the variation range of the resistance values Rb0 through Rm, and the thin film layer 13c is used in the variation range of the resistance value Rc0 through Rm. It is noted that those ranges of the resistance values are not limited to the aforementioned ranges; for example, the thin film layer 13a may be used in the variation range of the resistance values Ra0 through Ram, the thin film layer 13b may be used in the variation range of the resistance values Rb0 through Rbm, and the thin film layer 13c may be used in the variation range of the resistance value Rc0 through Rcm.

The hydrogen-gas concentration measuring device 20 measures the resistance values of the thin film layers 13a to 13c which the hydrogen detecting films 12a to 12c respectively have, and displays the hydrogen gas concentration or the like after determining the condition for measurement of the hydrogen gas concentration. The determination on the condition for measurement of the hydrogen gas concentration is carried out according to a flowchart illustrated in Fig. 5.

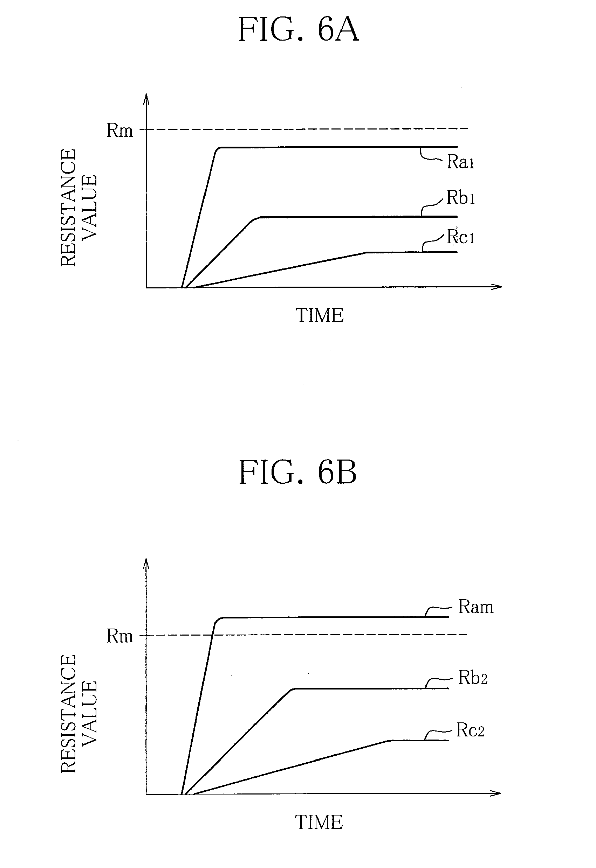

First, a description will be given of measurement of the hydrogen gas concentration when the hydrogen gas concentration is low. When the hydrogen gas concentration is low (concentration is assumed to be d1 (ppm)), i.e., none of resistance values Ra1, Rb1, Rc1 of the thin film layers 13a to 13c reach the limit resistance value Rm, as shown in Fig. 6A, the data processing unit 30 displays the hydrogen gas concentration on the basis of the resistance value Ra1 of the thin film layer 13a of the first hydrogen detecting film 12a.

Specifically, as shown in the flowchart of Fig. 5, the data processing unit 30 compares the resistance value Ra1 of the thin film layer 13a with the limit resistance value Rm. When the resistance value Ra1 is smaller than the limit resistance value Rm, the data processing unit 30 judges that none of the resistance values of the thin film layers 13a to 13c have reached the limit resistance value Rm (Y1 in step S1), and calculates and displays the hydrogen gas concentration on the basis of the resistance value Ra1 (step S4). That is, the hydrogen-gas concentration measuring device 20 can measure the hydrogen gas concentration with high accuracy in the variation range of the resistance value of the thin film layer 13a of the first hydrogen detecting film 12a (the range within Ra0 to Rm), as shown in Fig. 7.

The data processing unit 30 may calculate and display the hydrogen gas concentration on the basis of the resistance value Ra1 on condition that it is judged that all the relations of resistance value Ra1 < limit resistance value Rm, resistance value Rb1 < limit resistance value Rm, and resistance value Rc1 < limit resistance value Rm are satisfied.

When the hydrogen gas concentration is equal to or below the detectable limit of the hydrogen-gas concentration measuring device 20 (when the resistance values of the thin film layers 13a to 13c are lower limits Ra0, Rb0 and Rc0), the data processing unit 30 may display that the concentration is equal to or below the detectable limit.

When the condition that resistance value Ra1 < limit resistance value Rm is not satisfied (N1 in step S1), the data processing unit 30 calculates and displays the hydrogen gas concentration in the following procedures according to the flowchart of Fig. 5.

When resistance value Ra1 < limit resistance value Rm is not satisfied, i.e., when the hydrogen gas concentration is not low, the data processing unit 30 judges that the limit resistance value Rm has been reached in the thin film layer 13a of the first hydrogen detecting film 12a, and compare the resistance value Rb2 of the thin film layer 13b with the limit resistance value Rm in step S2 as illustrated in the flowchart of Fig. 5. When the resistance value Rb2 is lower than the limit resistance value Rm as shown in Fig. 6B, the data processing unit 30 judges that neither of the resistance values Rb2 and Rc2 of the thin film layers 13b and 13c has reached the limit resistance value Rm (Y2 in step S2), and calculates and displays the hydrogen gas concentration on the basis of the resistance value Rb2 (step S5). That is, when the hydrogen gas concentration is intermediate (concentration is assumed to be d2 (ppm)), the hydrogen-gas concentration measuring device 20 can measure the hydrogen gas concentration with high accuracy in the variation range of the resistance value of the thin film layer 13b of the second hydrogen detecting film 12b.

The thin film layer 13b is used in the range of the resistance value Rb0 to Rm, and the resistance value Ram has a relation Ram=2·Rbm with respect to the resistance value Rbm. Therefore, measurement of the hydrogen gas concentration in the range of Rb0 to about 0.5Rm (the broken line corresponding to the thin film layer 13b in Fig. 7) is carried out on the basis of the result of measuring the resistance value of the thin film layer 13a of the first hydrogen detecting film 12a.

When the condition that resistance value Rb2 < limit resistance value Rm is not satisfied (N2 in step S2), the data processing unit 30 calculates and displays the hydrogen gas concentration in the following procedures according to the flowchart of Fig. 5.

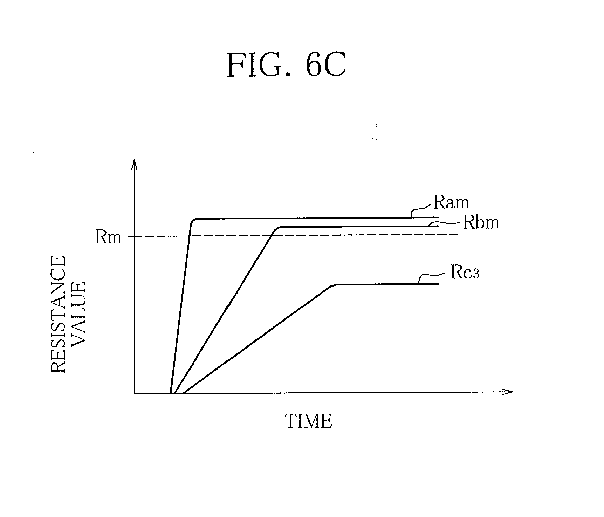

When the condition of resistance value Rb2 < limit resistance value Rm is not satisfied, i.e., when the hydrogen gas concentration is neither low nor intermediate, but is high (concentration is assumed to be d3 (ppm)), the data processing unit 30 judges that the limit resistance value Rm (limit) has been reached in the thin film layer 13b of the second hydrogen detecting film 12b, and compares the resistance value Rc3 of the thin film layer 13c with the limit resistance value Rm in step S3 as illustrated in the flowchart of Fig. 5. When the resistance value Rc3 is lower than the limit resistance value Rm as shown in Fig. 6C, the data processing unit 30 judges that only the resistance value of the thin film layer 13c has not reached the limit (Y3 in step S3), and calculates and displays the hydrogen gas concentration on the basis of the resistance value Rc3 (step S6).

When the condition that resistance value Rc3 < limit resistance value Rm is not satisfied (N3 in step S3), the data processing unit 30 displays that the hydrogen gas concentration exceeds the measurement limit (step S7), and returns the process to step S1 according to the flowchart of Fig. 5.

The thin film layer 13c is used in the range of the resistance value Rc0 to Rm, and the resistance values Ram, Rbm and Rcm have a relation Ram=2·Rbm=4·Rcm. As shown in fig. 7, therefore, measurement of the hydrogen gas concentration in the range of Rc0 to about 0.5Rm (the broken line corresponding to the thin film layer 13c in Fig. 7) is carried out on the basis of the result of measuring the resistance value of the thin film layer 13a of the first hydrogen detecting film 12a or the thin film layer 13b of the second hydrogen detecting film 12b.

Since the hydrogen gas concentration is detected in the above-described manner, as indicated by solid lines in Fig. 7, the hydrogen-gas concentration measuring device 20 measures the range of 0 to about 0.2 in the hydrogen-gas concentration measurement range of 0 to 1 with the first hydrogen detecting film 12a having the highest sensitivity, measures the range of about 0.25 to 0.5 with the second hydrogen detecting film 12b, and measures the range of about 0.5 to 1 with the third hydrogen detecting film 12c having the widest measurement range. If the hydrogen-gas concentration measuring device 20 processes the resistance value measuring results of the thin film layers 13a to 13c with the same resolution, i.e., if analog-to-digital conversion or the like is carried out with 10 bits, for example, the measurement accuracy at a low concentration can be improved, and the high accuracy of measurement can be maintained over a wide measurement range.

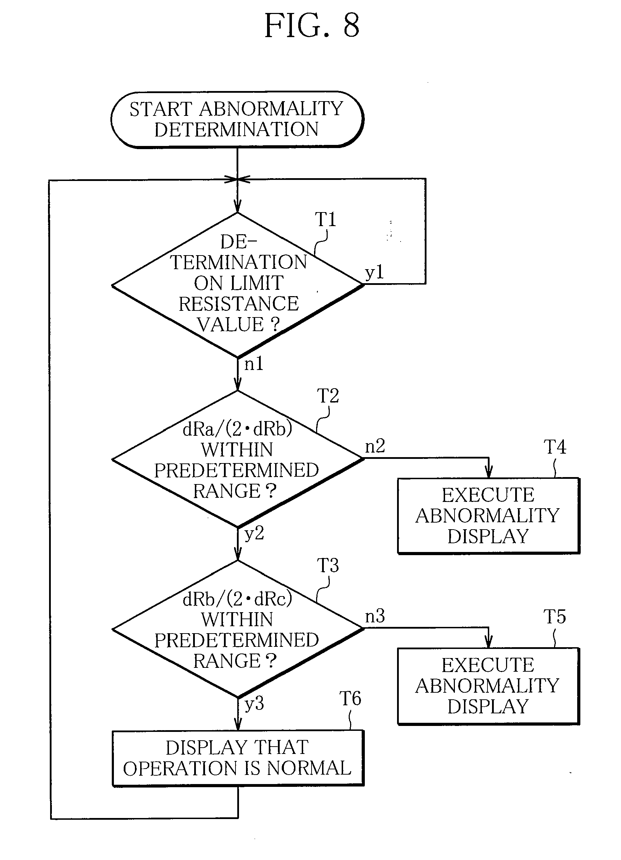

Next, referring to Fig. 8, judgment on an abnormality of the hydrogen-gas concentration sensor or the hydrogen-gas concentration measuring device by the hydrogen-gas concentration measuring device 20 will be explained. The hydrogen-gas concentration measuring device 20 measures the resistance values of the thin film layers 13a to 13c of the hydrogen detecting films 12a to 12c every unit time (dt (sec)). It is assumed that the resistance values of the thin film layers 13a, 13b and 13c in the steady state are Rad, Rbd and Rcd, respectively. In this case, if those resistance values satisfy the relation Rad=2·Rbd=4·Rcd, a change dRa in the resistance value of the thin film layer 13a, a change dRb in the resistance value of the thin film layer 13b and a change dRc in the resistance value of the thin film layer 13c in the period of the unit time dt (sec) satisfy the relation dRa=2·dRb=4·dRc.

The measurement controlling section 32 of the hydrogen-gas concentration measuring device 20 has a microprocessor and its program corresponding to a flowchart in Fig. 8, and executes the following process every unit time dt (sec).

The measurement controlling section 32 first determines whether none of the resistance values of the thin film layers 13a to 13c have reached the limit resistance value Rm (determination on limit resistance value in Step T1), and if the resistance value of any one of the thin film layers 13a to 13c has reached the limit resistance value Rm (y1 in step T1), the determination on limit resistance value in step T1 will be repeated.

When none of the resistance values of the thin film layers 13a to 13c have reached the limit resistance value Rm (n1 in step T1), the measurement controlling section 32 determines whether the value of dRa/(2·dRb) lies in a numerical range of, for example, 0.8 to 1.2. When the value of dRa/(2·dRb) does not lie in the numerical range (n2 in step T2), the measurement controlling section 32 displays the first hydrogen detecting film 12a and/or the second hydrogen detecting film 12b being abnormal, or occurrence of an abnormality in the hydrogen-gas concentration measuring device 20 on the display section 33 (step T4). When the value of dRa/(2·dRb) lies in the aforementioned range, on the other hand, the measurement controlling section 32 advances the process to step 3 (y2 in step T2).

In Step T3, the measurement controlling section 32 determines whether the value of dRb/(2·dRc) lies in a numerical range of, for example, 0.8 to 1.2. When the value of dRb/(2·dRc) does not lie in the range (n3 in step T3), the measurement controlling section 32 displays the second hydrogen detecting film 12b and/or the third hydrogen detecting film 12c being abnormal, or occurrence of an abnormality in the hydrogen-gas concentration measuring device 20 on the display section 33 (step T5). When the value of dRb/(2·dRc) lies in the aforementioned range (y3 in step T3), the measurement controlling section 32 displays that the operations of the hydrogen detecting films 12a, 12b, 12c and the operation of the hydrogen-gas concentration measuring device 20 are normal on the display section 33 (step T6), and returns the process to step 1.

In this manner, the hydrogen-gas concentration measuring device 20 can detect an abnormality in a hydrogen-gas concentration sensor or a hydrogen-gas concentration measuring device. If the numerical range used in abnormality determination is made narrower than 0.8 to 1.2, an abnormality can be determined more strictly. If the numerical range is made wider than 0.8 to 1.2, an abnormality can be determined more loosely.

As the upper limit and lower limit of the numerical range get closer to 1.0, an abnormality in a hydrogen-gas concentration sensor or a hydrogen-gas concentration measuring device can be detected more sensitively. If the upper limit and lower limit of the numerical range are set too close to 1.0, there arises a problem such that a difference in the reaction times of the thin film layers 13a to 13c with respect to the photocatalysis of the catalyst layers 14a to 14c is erroneously detected as being abnormal. In consideration of the difference in the reaction times, or the like, therefore, the numerical range can of course be changed as needed.

The present invention is not limited to the foregoing embodiment. For example, the hydrogen gas concentration can be measured with high accuracy in a wider measurement range by making the number of the hydrogen detecting films of the hydrogen-gas concentration sensor greater than that of the embodiment. Alternatively, the measurement accuracy, in particular, can be enhanced in a specific hydrogen gas concentration range by setting separate limit resistance values for the thin film layers of the respective hydrogen detecting films. As apparent from the above, the invention can be modified in a scope not departing from the gist of the invention. The hydrogen-gas concentration sensor is not limited to the type where the resistance value increases as the hydrogen gas concentration gets higher. In other words, the hydrogen-gas concentration sensor may have a resistance value which is high in a low concentration state, and becomes lower as the hydrogen gas concentration gets higher.

It is needless to say that the hydrogen gas concentration can be measured on the basis of the result of measuring a voltage drop in each thin film layer, instead of the resistance value of the thin film layer of each hydrogen detecting film. This is because a voltage drop in a thin film layer is the resistance value of the thin film layer multiplied by the current, and measurement of the resistance value of a thin film layer has substantially the same meaning as measurement of a voltage drop in a thin film layer. In short, according to the present invention, a voltage drop in a thin film layer has the same significance as the resistance value of a thin film layer.

Of course, the hydrogen gas concentration can be measured on the basis of the result of measuring the values of the currents flowing in the individual thin film layers with a predetermined voltage applied to the individual thin film layers, instead of the resistance values of the thin film layers of the individual hydrogen detecting films. This is because the value of the current flowing in a thin film layer is obtained by dividing the applied voltage by the resistance value of the thin film layer, and measurement of the value of the current flowing in a thin film layer has substantially the same meaning as measurement of the resistance value of a thin film layer. In short, according to the present invention, the value of the current flowing in a thin film layer has the same significance as the resistance value of a thin film layer.

## Claims

1. A hydrogen-gas concentration sensor, comprising:
a substrate; and
a plurality of hydrogen detecting films formed on the substrate, adjacent to one another, wherein
each of the plurality of hydrogen detecting films having a thin film layer formed on the substrate, and a catalyst layer formed on a surface of the thin film layer;
the catalyst layer exerts photocatalysis to hydrogenate the thin film layer reversibly when each of the hydrogen detecting films contacts a hydrogen gas contained in an atmosphere; and
electric resistance values of the respective thin film layers change reversibly with different sensitivities to a hydrogen gas concentration when the respective thin film layers are hydrogenated.

2. The hydrogen-gas concentration sensor according to claim 1, wherein each of the thin film layers is formed of a magnesium nickel alloy thin film layer or a magnesium thin film layer, and each of the catalyst layers is formed of palladium or platinum.

3. A hydrogen-gas concentration measuring device, comprising:
a hydrogen-gas concentration sensor for measuring a hydrogen gas concentration making use of photocatalysis;
a light source for irradiating the hydrogen-gas concentration sensor with light; and
a data processing unit for measuring a hydrogen gas concentration using the hydrogen-gas concentration sensor, wherein
the hydrogen-gas concentration sensor having a substrate, and a plurality of hydrogen detecting films formed on the substrate, adjacent to one another;
each of the plurality of hydrogen detecting films having a thin film layer formed on the substrate, and a catalyst layer formed on a surface of the thin film layer;
the catalyst layer exerts photocatalysis to hydrogenate the thin film layer reversibly when each of the hydrogen detecting films contacts a hydrogen gas contained in an atmosphere;
electric resistance values of the respective thin film layers change reversibly with different sensitivities to a hydrogen gas concentration when the respective thin film layers are hydrogenated; and
the data processing unit comprises a resistance measuring section for measuring an electric resistance value of each of the thin film layers of the plurality of hydrogen detecting films, and a measurement controlling section for measuring a hydrogen gas concentration on the basis of the respective electric resistance values of the thin film layers which are measured by the resistance measuring section, wherein
when none of the electric resistance values of the hydrogenated thin film layers have reached a predetermined limit resistance value, the measurement controlling section measures a hydrogen gas concentration on the basis of the electric resistance value of a thin film layer whose electric resistance value changes with a highest sensitivity to the hydrogen gas concentration, while when the hydrogenated thin film layers include a thin film layer whose electric resistance value has reached the predetermined limit resistance value, the measurement controlling section measures a hydrogen gas concentration on the basis of the electric resistance value of a thin film layer whose electric resistance value has not reached the limit resistance value.

4. The hydrogen-gas concentration measuring device according to claim 3, wherein each of the thin film layers is formed of a magnesium nickel alloy thin film layer or a magnesium thin film layer, and each of the catalyst layers is formed of palladium or platinum.

5. The hydrogen-gas concentration measuring device according to claim 3, wherein when the hydrogenated thin film layers include a thin film layer whose electric resistance value has reached the predetermined limit resistance value, the hydrogen-gas concentration measuring device measures a hydrogen gas concentration on the basis of the electric resistance value of a thin film layer having the electric resistance value that changes with a highest sensitivity to the hydrogen gas concentration among those thin film layers whose electric resistance values have not reached the predetermined limit resistance value.

6. The hydrogen-gas concentration measuring device according to claim 3, wherein
when the hydrogen-gas concentration measuring device detects a hydrogen gas, the resistance measuring section measures a variation per unit time in the electric resistance value of each of the thin film layers; and
the measurement controlling section compares the variations in at least two thin film layers with each other to acquire a value corresponding to a comparison result, and determines that the hydrogen-gas concentration sensor and/or the hydrogen-gas concentration measuring device has an abnormality when the value corresponding to the comparison result exceeds a predetermined range.
